(19) Europäisches Patentamt
European Patent Office
Office européen des brevets

(11) Publication number: 0 078 709
B1

(12) **EUROPEAN PATENT SPECIFICATION**

(45) Date of publication of patent specification: **01.10.86**

(51) Int. Cl.$^4$: **C 07 C 121/66**

(21) Application number: **82305830.0**

(22) Date of filing: **02.11.82**

# (54) Preparation of nitroaralkyl cyanides and derivatives thereof.

(30) Priority: **02.11.81 US 317321**
**25.08.82 US 411554**
**02.11.81 US 317322**
**16.09.82 US 418915**

(43) Date of publication of application:
**11.05.83 Bulletin 83/19**

(45) Publication of the grant of the patent:
**01.10.86 Bulletin 86/40**

(84) Designated Contracting States:
**AT BE CH DE FR GB IT LI LU NL SE**

(56) References cited:
**DE-A-1 913 741**
**US-A-2 080 057**

**JOURNAL OF MEDICINAL CHEMISTRY, VOL. 20, NO. 6, 1977; D.WE.DUNWELL ET AL.: "SYNTHESIS AND ANTIINFLAMMATORY ACTIVITY OF SOME 2-ARYL-6-BENZOXAZOLEACETIC ACID DERIVATIVES", PP. 797-801**

**ARZNEIMITTEL-FORSCHUNG (DRUG RES.), VOL. 23, NO. 8, 1973; G.NANNINI ET AL.: "NEW ANALGESIC-ANTIINFLAMMATORY DRUGS", PP. 1090-1100**

(73) Proprietor: **ETHYL CORPORATION**
**330 South Fourth Street P.O. Box 2189**
**Richmond Virginia 23219 (US)**

(72) Inventor: **Stahly, G. Patrick**
**6449 Peggy Street**
**Baton Rouge Louisiana 70808 (US)**
Inventor: **Stahly, Barbara Clack**
**6449 Peggy Street**
**Baton Rouge Louisiana 70808 (US)**

(74) Representative: **Bizley, Richard Edward et al**
**BOULT, WADE & TENNANT 27 Furnival Street**
**London EC4A 1PQ (GB)**

(56) References cited:
**EXPERIENTA, VOL. 29, NO. 8, 1979; R.W.J.CARNEY ET AL.: "A POTENT NON-STEROIDAL ANTI-INFLAMMATORY AGENT: 2-3-CHLORO-4(3-PYRROLINYL)PHENYL PROPIONIC ACID", P. 938**

**THE MERCK INDEX, 9TH EDITION, APPENDIX: APP-2, ABSTRACT A8, "FLUBIPROFEN"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 078 709 B1

**Description**

This invention relates to the preparation of nitroaralkyl cyanides and derivatives thereof.

There are known techniques of preparing profen-type pharmaceuticals and other materials having a relatively complex aryl group attached to the alpha-carbon of a substituted or unsubstituted acetic acid. For example, U.S. patents 3,755,427 (Adams et al.), 3,959,364 (Armitage et al.), and 4,278,516 (Zaiko et al.) disclose processes for converting various starting materials into flurbiprofen, i.e., 2 - (2 - fluoro - 4 - biphenylyl)propionic acid, and similar compounds having anti-inflammatory, analgesic, and anti-pyretic properties; Carney et al., "A Potent Non-Steroidal Anti-Inflammatory Agent: 2 - [3 - Chloro - 4 - (3 - pyrrolinyl) - phenyl]propionic Acid," *Experientia*, Vol. 29, page 938 (1973) teach, inter alia, the preparation of their anti-inflammatory agent—also known as pirprofen—via an ethyl 2 - (3 - chloro - 4 - nitrophenyl)propionate intermediate; U.S. Patent 4,239,901 (Rainer) shows that pyrazol - 1 - ylphenyl and pyrazolin - 1 - ylphenylacetic acids having anti-inflammatory properties can be synthesized from various intermediates, including polychloronitrophenylacetic acid esters; and it is known that indoprofen, i.e., 2 - [4 - (1,3 - dihydro - 1 - oxo - 2H - isoindol - 2 - yl)phenyl]propionic acid, and indobufen, i.e., 2 - [4 - (1,3 - dihydro - 1 - oxo - 2H - isoindol - 2 - yl)phenyl]butyric acid, can be prepared from the appropriate 2 - (4 - nitrophenyl) - alpha - alkylacetic acids.

Nitroarylacetic acids and their esters and nitriles, as well as the amino derivatives thereof, have been found to be particularly useful intermediates for the synthesis of these pharmaceuticals. However, in the past, a disadvantage of employing them as pharmaceutical intermediates has been the difficulty of preparing them by conventional techniques. Even the preferred procedures for preparing them have proven to be difficult, tedious, and time-consuming, as evidenced by Example 23 of U.S. Patent 3,868,391 (Carney et al. II) and Example 16 of Rainer, both of which show the use of days of refluxing to accomplish only a portion of their syntheses.

It would obviously be a welcome contribution to the art to provide a method of synthesizing nitrophenylalkyl cyanides and analogs and derivatives thereof in a simple and straightforward manner.

This invention aims to provide processes for preparing nitroaralkyl cyanides in moderate-to-good yield with high selectivity in a simple and straightforward manner. In addition, novel processes for preparing derivatives of the cyanides are provided.

The invention provides a process comprising reacting a nitroaromatic compound with a cyanide compound of the formula

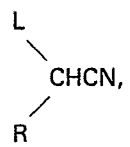

wherein L is chlorine or bromine and R is alkyl, in a substantially anhydrous aprotic solvent and in the presence of a base so that the nitrile undergoes a nucleophilic substitution on an unsubstituted ring carbon of the nitroaromatic compound during which the chlorine or bromine L functions as a leaving group, thereby forming a nitroarylacetonitrile and, when appropriate, converting the nitroarylacetonitrile to a desired derivative thereof.

The invention also provides the novel compound 2 - (3 - fluoro - 4 - nitrophenyl) propionitrile, which is useful as an intermediate for the preparation of flurbiprofen.

Nitroaromatic compounds utilizable in the practice of the invention include a variety of such compounds—the chief requirements for their utility being that (1) they bear at least one nitro substituent on an aromatic ring, (2) they contain at least one replaceable hydrogen on an aromatic ring to which a nitro group is attached, and (3) they be devoid of substituents which would interfere with the desired nucleophilic substitution reaction, e.g., substituents bearing an acidic proton, such as —$NH_2$, —OH, —SH, —NHR, etc.

Thus, the utilizable nitroaromatic compounds include compounds having one or more simple or fused aromatic rings containing five or six members and either bearing no substituents other than nitro substituents or also bearing any of a variety of inert substituents, i.e., substituents that do not interfere with the desired nucleophilic substitution reaction, such as halo, alkyl, alkoxy, alkylmercapto, trifluoromethyl, dialkylamino, dialkanoylamino, cyano, dialkylcarbamoyl, alkylsulfonyl, dialkylsulfamoyl, alkoxyalkyl, haloalkyl, cycloalkyl, halocycloalkyl, etc.—any alkyl chains in the substituents generally being lower alkyl chains. When the nitroaromatic compound contains more than one ring, any such inert substituent may be on the same ring as the ring bearing a nitro substituent and/or on a ring which is directly or indirectly attached to the ring bearing a nitro substituent.

When the aromatic ring bearing the required nitro substituent is a six-membered ring, there will be at least one replaceable hydrogen in a position para or ortho to the carbon bearing the nitro substituent; and it is preferred that there be a replaceable hydrogen in the para position. Nitroaromatic compounds having a five-membered ring should have a replaceable hydrogen on a carbon adjacent to, or separated by two ring atoms from, the carbon bearing the nitro substituent.

In accordance with one embodiment of the invention, the nitroaromatic compound is a compound which is devoid of halogen on the aromatic ring bearing the required nitro group. Illustrative of such compounds are heterocyclic compounds which preferably contain five or six-membered rings having aromatic character, such as nitropyridine-N-oxide, 5-nitroisoquinoline, 5- and 6-nitroquinolines, 2-nitrothiophene, etc.; fused-ring aromatic compounds, such as the 1- and 2-nitronaphthalenes, etc.; aromatic compounds containing a plurality of simple rings, such as the 2-, 3-, and 4-nitrobiphenyls, the 2-, 3-, and 4-benzylnitrobenzenes, 2-nitrodiphenyl ether, etc.; and aromatic compounds containing a single simple ring, such as nitrobenzene, 2-methylnitrobenzene, the 2,3-, 2,5-, and 3,5-dimethylnitrobenzenes, the 2,4- and 2,6-diethylnitrobenzenes, 3,4-dibutylnitrobenzene, the 1,2- and 1,3-dinitrobenzenes, 2,6-dinitrotoluene, the 1,2,3- and 1,2,4-trinitrobenzenes, 2 - nitro - N,N - diethylaniline, 4 - nitro - N - ethylacetanilide, 2 - nitrobenzylcyanide, 2-nitrophenyl acetate, etc.

In accordance with another embodiment of the invention, the nitroaromatic compound is a compound which bears at least one halo substituent on the aromatic ring bearing the required nitro group. Exemplary of such compounds are the 2-, 3-, and 4-chloronitrobenzenes; the 2,3-, 2,4-, 2,5-, 2,6-, 3,4-, and 3,5-dichloronitrobenzenes; the various trichloronitrobenzenes; the corresponding fluoro, bromo, and iodo compounds; the various dimethyl-, diethyl-, and dibutylnitrobenzenes, nitrobiphenyls, benzylnitrobenzenes, nitronaphthalenes, di- and trinitrobenzenes, nitro - N,N - diethylanilines, nitrodiphenyl esters, nitro - N - ethylacetanilides, nitrobenzylcyanides, nitrophenyl acetates, nitro - pyridine - N - oxides, nitroquinolines, nitroisoquinolines, nitrothiophenes, and the like bearing one or more ar-chloro, fluoro, bromo, or iodo substituents and containing at least one replaceable hydrogen in an appropriate position.

In some cases, polynitroaromatic reactants may undergo substitution reactions whereby one of the nitro groups is replaced by the nitrile reactant. Therefore, the possibility of this competitive reaction should be kept in mind when selecting a polynitroaromatic reactant for use in the process.

In each of the aforementioned embodiments of the invention, the preferred nitroaromatic compounds are nitrobenzenes having a replaceable hydrogen in the position para to the nitro group, since the nucleophilic substitution reaction of the invention tends to be highly selective on the para position, and the use of such compounds therefore leads to the production of nitrophenylacetonitriles which are ideally suited for the synthesis of anti-inflammatory agents of the type described in the aforementioned references. Even more preferred in the case of the halonitrobenzenes are such nitrobenzenes having a halo substituent in a position ortho to the nitro group. Halonitrobenzenes which are especially preferred are 2-chloronitrobenzene and 2 - fluoronitrobenzene which are readily converted with highly selectivity into such products as 2 - [3 - chloro - 4 - (3 - pyrrolinyl)phenyl]propionic acid, 2 - (2 - fluoro - 4 - biphenylyl)propionic acid, and related anti-inflammatory agents. A non-halogenated nitroaromatic compound that is particularly preferred is nitrobenzene, which is readily converted with high selectivity into pharmaceutically-active agents such as 2 - [4 - (1,3 - dihydro - 1 - oxo - 2H - isoindol - 2 - yl)phenyl] - propionic acid, 2 - [4 - (1,3 - dihydro - 1 - oxo - 2H - isoindol - 2 - yl)phenyl]butyric acid, and analogs thereof.

The cyanide compounds that can be used in the practice of the invention are compounds, which may be represented by the formula:

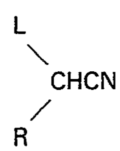

wherein L is chlorine or bromine and R is an alkyl group which most preferably contains up to about 10 carbons.

A few examples of the cyanide compounds that can be used in the practice of the invention are 2 - chloropropionitrile, 2 - chlorobutyronitrile, 2 - chlorovaleronitrile, 2 - chlorocapronitrile, 2 - chloro - 3,3 - dimethylbutyronitrile, and the corresponding bromo compounds, 2 - Chloropropionitrile and 2 - bromopropionitrile are particularly preferred.

Illustrative aprotic solvents which may be employed in the process of the invention include ethers such as diethyl ether, dibutyl ether, 1-ethoxyhexane, tetrahydrofuran, 1,4-dioxane, 1,3-dioxolane, diglyme, 1,2-diethoxyethane, and anisole; tertiary amines such as pyridine, N-ethylpiperidine, triethyl amine, tributyl amine, N,N - diphenyl - N - methyl amine and N,N - dimethylaniline. However, the preferred aprotic solvents are dipolar aprotic solvents such as dimethyl sulfoxide, N,N - dimethylformamide, N,N - dimethylacetamide, dimethyl sulfone, tetramethylene sulfone and, N - methylpyrrolidone.

Bases useful in the practice of the invention include all bases strong enough to activate the nitrile reactant, e.g., alkaline earth metal compounds such as calcium oxide, calcium hydride, calcium hydroxide, barium oxide, barium hydroxide, magnesium hydroxide, zinc hydroxide, etc. However, the base is preferably an alkali metal compound, e.g., an organoalkali metal compound, alkali metal hydride, alkali metal hydroxide, alkali metal oxide, alkali metal amide, or alkali metal alcoholate, such as butyllithium, phenyllithium, ethylsodium, amylsodium, butylpotassium, benzylpotassium, sodium dimesylate (i.e., the sodium salt of diethylsulfoxide), sodium hydride, potassium hydride, sodium hydroxide, potassium

hydroxide, sodium oxide, potassium oxide, sodium amide, potassium amide, lithium diisopropylamide, sodium methoxide, potassium t-butoxide, the sodium salt of the monomethylether of ethylene glycol, sodium phenoxide, and the like. Ordinarily the use of sodium hydride or potassium hydride will be found most convenient and economical.

Use of an alkali metal compound as the base permits the alternatives of using the alkali metal compound alone or in conjunction with a phase transfer catalyst, such as a quaternary ammonium salt, ethylene glycol, or a suitable crown ether. When a phase transfer catalyst is employed (1) the alkali metal compound may be any of the alkali metal compounds generically or specifically indicated above, although the type of alkali metal compound being used determines the type of crown ether that is preferably utilized—lithium bases generally calling for the use of a 12-crown-4 ether, sodium bases usually calling for the use of a 15-crown-5-ether, and potassium bases generally calling for the use of an 18-crown-6-ether, and (2) the reaction medium may be any of the aprotic solvents mentioned above, or it may be an inert liquid hydrocarbon such as benzene, toluene, xylene, hexane, heptane, isooctane, or the like.

When an alkali metal hydride, especially a highly pure alkali metal hydride, is employed as the base, it is desirable to include a small amount of a transfer agent such as water, alcohol, or the like in the system. It is believed that the transfer agent activates the hydride by reacting therewith to form a small amount of the alkali metal hydroxide or alcoholate.

The nitroaralkyl cyanide synthesis of the invention is conducted in a substantially anhydrous reaction system, and accordingly, except when a small amount of water (which is itself consumed by reaction with the alkali metal hydride) is employed as a transfer agent, the components of the reaction system should be brought together and maintained under a dry inert atmosphere. Thus, while it is possible to conduct the process in the presence of air, it is desirable to maintain the reaction system under an atmosphere of dry nitrogen or the like. Since the reaction itself is normally an exothermic reaction, with its initiation readily ascertainable by noting the exotherm produced, the reactants are ordinarily brought together at ambient temperatures, although the temperature may be raised or lowered to suit the needs of the occasion if desired.

The nitroaromatic compound and cyanide compound may be used in amounts such as to provide a stoichiometric excess of either of the reactants or the stoichiometric amount of each. However, when a stoichiometric excess of the nitroaromatic compound is employed, the quantity of product obtainable will be limited by the quantity of cyanide compound used, so it is desirable to utilize a stoichiometric excess of the cyanide compound. The amount of base employed is preferably such as to provide at least two molar equivalents of base per mol of nitroaromatic compound, since the use of smaller amounts—although permitting the reaction to occur—makes the base the limiting reagent.

The mode of addition of the ingredients of the reaction system is not particularly critical. Accordingly, it is convenient to add the nitroaromatic compound to a mixture of the other materials, add the base to a mixture of the other materials, add the reactants to a mixture of the base and aprotic solvent, introduce all four ingredients simultaneously into the reaction zone, or the like. Since the reaction ordinarily proceeds very rapidly, long reaction times are not required. The reaction will usually be completed within a matter of minutes or a few hours at ambient temperatures.

When derivatives of the nitroaralkyl cyanides are desired, they may be prepared by employing conventional techniques to convert to the desired derivatives the nitroaralkyl cyanides made in accordance with the present invention. Thus, for example:

(A) 2 - (4 - nitrophenyl)propionitrile synthesized by the process of the invention may be hydrolyzed to 2 - (4 - nitrophenyl)propionic acid, which in turn may be hydrogenated to 2 - (4 - aminophenyl)propionic acid, reacted with phthalic anhydride to form 2 - (4 - phthalimidophenyl)propionic acid, and reduced to indoprofen,

(B) 2 - (4 - nitrophenyl)propionitrile synthesized by the process of the invention may be hydrogenated to 2 - (4 - amino - phenyl)propionitrile, hydrolyzed to 2 - (4 - aminophenyl)propionic acid, reacted with phthalic anhydride to form 2 - (4 - phthalimidophenyl)propionic acid, and reduced to indoprofen,

(C) 2 - (4 - nitrophenyl)propionitrile synthesized by the process of the invention may be hydrogenated to 2 - (4 - aminophenyl)propionitrile, reacted with phthalic anhydride to form 2 - (4 - phthalimidophenyl)propionitrile, and hydrolyzed and reduced (in either order) to indoprofen,

(D) 2 - (4 - nitrophenyl)butyronitrile synthesized by the process of the invention may be subjected to the same reactions to prepare indobufen or indobufen intermediates,

(E) 2 - (3 - chloro - 4 - nitrophenyl)propionitrile synthesized by the process of the invention may be reduced to 2 - (4 - amino - 3 - chlorophenyl)propionitrile, reacted with a 1,4 - dihalo - 2 - butene to form 2 - [3 - chloro - 4 - (3 - pyrrolinyl)phenyl]propionitrile, and then converted into 2 - [3 - chloro - 4 - (3 - pyrrolinylphenyl] - propionic acid, and

(F) 2 - (3 - fluoro - 4 - nitrophenyl)propionitrile synthesized by the process of the invention may be reduced to 2 - (4 - amino - 3 - fluorophenyl)propionitrile, converted into (2 - fluoro - 4 - biphenylyl)propionitrile by means of a Gomberg-Bachmann reaction with benzene, and then converted into 2 - (2 - fluoro - 4 - biphenylyl)propionic acid.

The particular conventional techniques used to convert the nitroaralkyl cyanides into their various derivatives are not critical. It may sometimes be desirable to use certain particular techniques for the preparation of the derivatives, e.g., (a) the reduction and/or hydrolysis techniques taught in March,

*Advanced Organic Chemistry,* McGraw-Hill, New York, 1977, pages 809—10, 1125—6, and the references cited therein, (b) the Gomberg-Bachmann techniques taught in March, pages 653—4, and in *Organic Reactions,* Vol. 2, page 224 (1944); Journal of the *American Chemical Society,* Vol. 46, page 2339 (1924); *Chemical Rev.* Vol. 57, page 77 (1957); and *Journal of the Chemical Society,* Vol. D 1971, page 411, and (c) the techniques taught in Adria Laboratories, Inc.'s NDA on Indoprofen Capsules, Section No. 8(c), pages 2—11, which is on file with the Federal Drug Administration. However, the overall processes for preparing the derivatives are simplified and made more efficient and economical by the present simplification of the synthesis of the nitroaralkyl cyanides regardless of the particular techniques used to convert them into their various derivatives.

The invention includes the preparation of pharmaceutical or veterinary formulations comprising those of said derivatives which have therapeutic activity, in each case said derivative being prepared by a process of the invention and formulated for pharmaceutical or veterinary use, respectively.

As indicated, the present invention is particularly advantageous in providing a readier and more economical route to the synthesis of pharmaceuticals and other chemical products that can be prepared from nitroaralkyl cyanides. Such products include, not only those mentioned above, but a variety of products, such as products disclosed in U.S. Patents 3,641,040, 3,657,230, 3,767,805, 3,868,391, 3,936,467, 3,993,763, 3,997,669, 4,010,274, 4,118,504, 4,126,691, 4,163,788, and 4,239,901.

The following Examples are given to illustrate the invention.

Example I

Into a flame dried flask under nitrogen was placed 1.3 grams (0.026 mol) of NaH (50% dispersion in mineral oil). This was washed twice with 10 ml portions of petroleum ether (b.p. 35—60°C) and dried in a nitrogen stream. Then 25 ml of N,N - dimethylformamide (DMF; dried over 3 Angstrom molecular sieves) was added followed by dropwise addition (over 20 minutes) of a solution of 2.2 ml (0.021 mol) of 2-fluoronitrobenzene and 1.9 ml (0.023 mol) of 2-chloropropionitrile in 10 ml of DMF. The mixture became red and hot during the dropwise addition. A small portion of the reaction mixture was worked up by partitioning between 1N HCl and diethyl ether, and analysis of the ether layer by gas chromatography (GC) indicated some starting material had not reacted. A second (0.40 g, 0.008 mol) and third (0.80 g, 0.017 mol) portion of 50% NaH were added so that workup of a reaction mixture sample followed by GC analysis indicated that no starting material remained. The reaction mixture was poured into 250 ml of 1N HCl and extracted with six 200 ml portions of ether. The ether layers were combined, dried ($MgSO_4$), and concentrated to give a black oil which was adsorbed on 15 g of Silica Gel 60 (230—400 mesh) and loaded on a column of 150 g Silica Gel 60 packed in 40% $CH_2Cl_2$/60% petroleum ether (bp 35—60°C). Elution with the same solvent mixture afforded four fractions which, by GC area %, contain 1.8 g (44%) of 2 - (3 - fluoro - 4 - nitrobenzene)propionitrile. This compound was characterized by NMR, IR, and mass spectrometry.

Example II

A slurry of 240 mg (5.0 mmols) of NaH (50% in mineral oil) in 2 ml of pyridine was treated dropwise, under nitrogen, with 0.26 ml (0.35 g, 2.5 mmols) of 2-fluoronitrobenzene followed by 0.22 ml (0.23 g, 2.6 mmols) of 2-chloropropionitrile. The purple reaction mixture was stirred at room temperature under nitrogen for 15 minutes, then was poured into an equal volume of 5% HCl and extracted with an equal volume of $CH_2Cl_2$. The organic phase was shown by gas chromatography-mass spectral analysis (GC-MS) to contain 2 - (3 - fluoro - 4 - nitrobenzene)propionitrile.

Example III

A solution of 0.61 g (3.1 mmol) of 2 - (3 - fluoro - 4 - nitrobenzene)propionitrile in 10 ml of absolute ethanol was treated with 0.03 g of 7% palladium on carbon and hydrogenated at 45 psi of hydrogen (Parr apparatus) for 1 hour. The reaction mixture was filtered and concentrated to give 0.54 g of an oil which darkened on standing. A portion of this oil was purified on 1 mm silica gel plates (developed with $CH_2Cl_2$) to give 2 - (4 - amino - 3 - fluorobenzene)propionitrile, which was characterized by NMR, IR, and mass spectrometry.

Example IV

A solution of 1.2 g (6.2 mmol) of 2 - (3 - fluoro - 4 - nitrobenzene)propionitrile in 24 ml of absolute ethanol was treated with 0.06 g of 7% palladium on carbon and hydrogenated at 40—45 psi hydrogen pressure (Parr apparatus) for 1 hour. The reaction mixture was filtered and the filtrate was shown to contain, by GC analysis (area percent) 99% 2 - (4 - amino - 3 - fluorobenzene)propionitrile. Removal of the solvent in a rotary evaporator gave 1.1 gram of a yellow oil which quickly darkened on standing.

Example V

A solution of 25 mg (0.15 mmol) of 2 - (4 - amino - 3 - fluorobenzene)propionitrile, 0.2 ml of benzene, and 0.03 ml (0.25 mmol) of isoamyl nitrite was heated at reflux for 1.5 hours. GC analysis (area percent) of the reaction mixture indicated the presence of 23% unreacted starting material and 60% of a product which was identified by GC-MS to be 2 - (2 - fluoro - 4 - biphenylyl) - propionitrile.

Example VI

To a slurry of 0.50 g (10 mmols) of NaH (50% dispersion in mineral oil) in 2 ml of N,N - dimethylformamide (DMF, dried over 3 Angstrom molecular sieves) under nitrogen was added dropwise, over a period of 10 minutes, a solution of 0.60 ml (5.1 mmols) of 2-chloronitrobenzene and 0.42 ml (5.1 mmols) of 2-chloropropionitrile in 1 ml of DMF. During the addition, the mixture became purple and an exotherm was observed. The reaction mixture was stirred under nitrogen for 15 minutes, poured into 30 ml of 1N HCl and extracted with four 30 ml portions of diethyl ether. The organic layers were combined, dried using $MgSO_4$, and concentrated to give 1.2 g of a black oil. Preparative thin layer chromatography of 0.20 g of this oil (one 2 mm silica gel plate developed with 50% $CH_2Cl_2$/50% petroleum ether) afforded 0.088 gram of 2 - (3 - chloro - 4 - nitrobenzene)propionitrile which was characterized by means of NMR and mass spectrometry.

Example VII

Into a flask under nitrogen was placed 4.0 g of 60% sodium hydride in mineral oil (0.10 mole). The sodium hydride was washed with three 10 ml portions of petroleum ether (b.p. 35—60°C) and was slurried in 50 ml of N,N - dimethylformamide (DMF). A solution of 12.3 g of nitrobenzene (0.10 mole) and 9.0 g of 2-chloropropionitrile (0.10 mole) in 10 ml of DMF was added dropwise to the slurry. An ice water bath was applied to the mixture periodically so that the temperature did not exceed 45°C. After the addition was complete (30 minutes) the purple mixture was allowed to react for 30 minutes and was poured into 200 ml of cold 10% HCl. The aqueous mixture was extracted with three 100 ml portions of diethyl ether and the ether layers were combined, dried ($MgSO_4$), and concentrated to give a dark oil. Excess nitrobenzene and DMF were removed from this oil at 50°C (at 1 mm pressure) and the residue was chromatographed on a column of 400 g of silica gel which was eluted with dichloromethane. A fraction was collected containing 2.9 g of 2 - (4 - nitrobenzene)propionitrile (16.5% yield).

Example VIII

Into a flame dried flask under nitrogen was placed 500 mg of 60% sodium hydride in mineral oil (12.5 mmole). The sodium hydride was washed with three 5 ml portions of petroleum ether (b.p. 35—60°C) and was slurried in 4 ml of N,N - dimethylformamide (DMF). One drop of a solution of 770 mg of nitrobenzene (6.25 mmole) and 600 mg of 2-chloropropionitrile (6.70 mmole) in 1 ml of DMF was added to the sodium hydride slurry to give a deep purple solution. After one minute the mixture was placed in an ice water bath and the rest of the reactant solution was added dropwise. The resulting mixture was stirred at 0°C for 15 minutes and was poured into 50 ml of 1N HCl. The aqueous mixture was extracted with three 40 ml portions of diethyl ether and the ether layers were combined, dried ($MgSO_4$), and concentrated to give 880 mg of black oil. Purification of 208 mg of this oil on one 2 mm silica gel plate eluted with 50% dichloromethane/50% petroleum ether afforded 51.2 mg (20% yield) of 2 - (4 - nitrobenzene)propionitrile.

Example IX

Into a flame dried flask under nitrogen was placed 176 mg of potassium *tert*-butoxide (1.57 mmoles), 23 mg of dibenzo 18-crown-6 ether (0.064 mmole), and 1.0 ml of toluene. While this mixture was vigorously stirred in a room temperature water bath a solution of 106 mg of 1,3-dinitrobenzene (0.631 mmole) and 71 mg of 2-chloropropionitrile (0.79 mmole) in 0.5 ml of toluene was added dropwise. The resulting purple mixture was stirred for 15 minutes and poured into 20 ml of 1N HCl. The aqueous mixture was extracted with three 20 ml portions of diethyl ether and the ether layers were combined, dried ($MgSO_4$), concentrated, and placed on one 2 mm silica gel TLC plate. One development with 50% petroleum ether (pp 35—60°C)/50% dichloromethane afforded 10 mg of 1,3-dinitrobenzene (9% recovery) and 25 mg of 2 - (2,4 - di - nitrobenzene)propionitrile (18% yield).

The preceding Examples demonstrate the utility of alpha-haloalkyl cyanides (i.e. alpha-Cl or Br, alpha-alkyl acetonitrile) in the preparation of nitroaralkyl cyanides. The following example shows that similar results are not achieved when an acetonitrile having only one alpha-substituent is substituted for the alpha-Cl or Br, alpha-alkyl acetonitriles.

Comparative Example

Into a flame dried flask under nitrogen was placed 500 mg of 60% sodium hydride in mineral oil (12.5 mmole). The sodium hydride was washed with three 5 ml portions of petroleum ether (b.p. 35—60°C), slurried in 4 ml of N,N-dimethylformamide (DMF), and cooled in an ice water bath. A solution of 770 mg of nitrobenzene (6.25 mmole) and 500 mg of chloroacetonitrile (6.62 mmole) in 1 ml of DMF was added dropwise. The resulting brown mixture was stirred at 0°C for 15 minutes and was poured into 50 ml of 1N HCl. The aqueous mixture was extracted with three 40 ml portions of diethyl ether and the ether layers were combined, dried ($MgSO_4$), and concentrated to give 1.02 g of black oil. Thin layer chromatographic analysis of this oil revealed no substitution product; only nitrobenzene and an immobile brown spot were observed.

**Claims**

1. A process for preparing a nitroaralkyl cyanide which comprises reacting a nitroaromatic compound with a cyanide compound of the formula

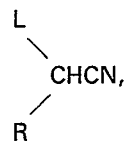

wherein L is chlorine or bromine and R is alkyl, in a substantially anhydrous aprotic solvent and in the presence of a base so that the cyanide undergoes a nucleophilic substitution on an unsubstituted ring carbon of the nitroaromatic compound during which the chlorine or bromine L functions as a leaving group.

2. A process as claimed in claim 1, wherein the nitroaromatic compound is devoid of halogen on the aromatic ring carrying the nitro group.

3. A process as claimed in claim 1, wherein the nitroaromatic compound is a halonitroaromatic compound having a halo substituent on the aromatic ring carrying the nitro group.

4. A process as claimed in claim 2 or claim 3, wherein the nitroaromatic compound is a mononitrobenzene having an unsubstituted position para to the nitro group.

5. A process as claimed in claim 4, wherein the mononitrobenzene is nitrobenzene or an o-halonitrobenzene.

6. A process as claimed in claim 1, wherein the nitroaromatic compound is a chloronitrobenzene, preferably o-chloronitrobenzene.

7. A process as claimed in claim 1, wherein the nitroaromatic compound is a fluoronitrobenzene, preferably o-fluoronitrobenzene.

8. A process as claimed in any one of claims 1 to 7, wherein R is a $C_1$—$C_{10}$ alkyl group.

9. A process as claimed in any one of claims 1 to 8, wherein the solvent is a dipolar aprotic solvent.

10. A process as claimed in any one of claims 1 to 9, wherein the base is an alkali metal compound, preferably sodium hydride or potassium hydride.

11. A process as claimed in any one of claims 1 to 10, wherein the nitroaralkyl cyanide is a 2 - (4 - nitro - phenyl)acetonitrile, a 2 - (4 - nitrophenyl)propionitrile, or a 2 - (3 - halo - 4 - nitrophenyl)propionitrile.

12. 2 - (3 - fluoro - 4 - nitrophenyl)propionitrile.

13. A process for preparing 2 - [4 - (1,3 - dihydro - 1 - oxo - 2H - isoindol - 2 - yl)phenyl]propionic acid comprising either:

(a) preparing 2 - (4 - nitrophenyl)propionitrile by a process as claimed in claim 11 and converting the prepared 2 - (4 - nitrophenyl)propionitrile to 2 - (4 - aminophenyl) - propionic acid either by hydrolyzing the 2 - (4 - nitrophenyl) - propionitrile to 2 - (4 - nitrophenyl)propionic acid and reducing said acid, or by reducing the 2 - (4 - nitrophenyl) - propionitrile to 2 - (4 - aminophenyl)propionitrile and hydrolyzing the 2 - (4 - aminophenyl)propionitrile, in either case thereafter reacting the 2 - (4 - aminophenyl)propionic acid with phthalic anhydride to form 2 - (4 - phthalimidophenyl)propionic acid and reducing the 2 - (4 - phthalimidophenyl)propionic acid; or

(b) preparing 2 - (4 - nitrophenyl)propionitrile by a process as claimed in claim 11 and reducing the prepared 2 - (4 - nitrophenyl)propionitrile to 2 - (4 - aminophenyl) - propionitrile, reacting the 2 - (4 - aminophenyl)propionitrile with phthalic anhydride to form 2 - (4 - phthalimidophenyl) - propionitrile, and converting the 2 - (4 - phthalimidophenyl) - propionitrile into 2 - [4 - (1,3 - dihydro - 1 - oxo - 2H - isoindol - 2 - yl)phenyl]propionic acid either by hydrolyzing the 2 - (4 - phthalimidophenyl)propionitrile to 2 - (4 - phthalimidophenyl)propionic acid and reducing said acid, or by reducing the 2 - (4 - phthalimidophenyl)propionitrile to 2 - [4 - (1,3 - dihydro - 1 - oxo - 2H - isoindol - 2 - yl)phenyl] - propionitrile followed by hydrolysis.

14. A process for preparing 2 - [3 - chloro - 4 - (3 - pyrrolinyl)phenyl]propionic acid comprising preparing 2 - (3 - chloro - 4 - nitrophenyl)propionitrile by a process as claimed in claim 11 and reducing the 2 - (3 - chloro - 4 - nitrophenyl)propionitrile to 2 - (4 - amino - 3 - chlorophenyl) - propionitrile, reacting the 2 - (4 - amino - 3 - chlorophenyl) - propionitrile with a 1,4 - dihalo - 2 - butene to form 2 - [3 - chloro - 4 - (3 - pyrrolinyl)phenyl]propionitrile, and hydrolyzing the 2 - [3 - chloro - 4 - (3 - pyrrolinyl)phenyl] - propionitrile.

15. A process for preparing 2 - (2 - fluoro - 4 - biphenylyl)propionic acid comprising preparing 2 - (3 - fluoro - 4 - nitrophenyl)propionitrile by a process as claimed in claim 11, reducing the 2 - (3 - fluoro - 4 - nitrophenyl)propionitrile to 2 - (4 - amino - 3 - fluorophenyl)propionitrile and converting the prepared 2 - (4 - amino - 3 - fluorophenyl)propionitrile into 2 - (2 - fluoro - 4 - biphenylyl) - propionitrile by means of a Gomberg-Bachmann reaction with benzene, and hydrolyzing the 2 - (2 - fluoro - 4 - biphenylyl)propionitrile.

16. A process for preparing a pharmaceutical or veterinary formulation comprising as an active

compound 2 - (2 - fluoro - 4 - biphenylyl)propionic acid, 2 - [3 - chloro - 4 - (3 - pyrrolinyl)phenyl]propionic acid or 2 - [4 - (1,3 - dihydro - 1 - oxo - 2H - isoindol - 2 - yl)phenyl]propionic acid, the process comprising preparing the active compound by a process as claimed in claim 15, claim 14 or claim 13, respectively, and formulating the active compound for pharmaceutical or veterinary use.

17. The use of a process as claimed in claim 15, claim 14 or claim 13, respectively, to prepare 2 - (2 - fluoro - 4 - biphenylyl)propionic acid, 2 - [3 - chloro - 4 - (3 - pyrrolinyl)phenyl]propionic acid or 2 - [4 - (1,3 - dihydro - 1 - oxo - 2H - isoindol - 2 - yl)phenyl]propionic acid for use as a pharmaceutical.

**Patentansprüche**

1. Verfahren zur Herstellung eines Nitroaralkylcyanids, dadurch gekennzeichnet, daß man eine nitroaromatische Verbindung mit einer Cyanid-Verbindung der allgemeinen Formel

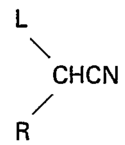

in welcher L Chlor oder Brom und R Alkyl bedeutet, in einem im wesentlichen wasserfreien aprotischen Lösungsmittel und in Gegenwart einer Base so umsetzt, daß das Cyanid in einer nukleophilen Substitution, während welcher das Chlor oder das Brom L eine austretende Gruppe bedeutet, an einen nichtsubstituierten Ringkohlenstoff der nitroaromatischen Verbindung tritt.

2. Verfahren nach Anspruch 1, dadurch gekennzeichnet, daß die nitroaromatische Verbindung an dem die Nitrogruppe tragenden aromatischen Ring kein Halogen enthält.

3. Verfahren nach Anspruch 1, dadurch gekennzeichnet, daß die nitroaromatische Verbindung eine halogen-nitroaromatische Verbindung mit einem Halogensubstituent an dem die Nitrogruppe tragenden aromatischen Ring ist.

4. Verfahren nach einem der Ansprüche 2 oder 3, dadurch gekennzeichnet, daß die nitroaromatische Verbindung ein in para-Stellung zu der Nitrogruppe nichtsubstituiertes Mononitrobenzol ist.

5. Verfahren nach Anspruch 4, dadurch gekennzeichnet, daß das Mononitrobenzol Nitrobenzol oder ein o-Halogennitrobenzol ist.

6. Verfahren nach Anspruch 1, dadurch gekennzeichnet, daß die nitroaromatische Verbindung ein Chlornitrobenzol, bevorzugt o-Chlornitrobenzol, ist.

7. Verfahren nach Anspruch 1, dadurch gekennzeichnet, daß die nitroaromatische Verbindung ein Fluornitrobenzol bevorzugt o-Fluornitrobenzol, ist.

8. Verfahren nach einem der Ansprüche 1 bis 7, dadurch gekennzeichnet, daß R eine $C_1$-bis $C_{10}$-Alkylgruppe ist.

9. Verfahren nach einem der Ansprüche 1 bis 8, dadurch gekennzeichnet, daß das Lösungsmittel ein dipolares aprotisches Lösungsmittel ist.

10. Verfahren nach einem der Ansprüche 1 bis 9, dadurch gekennzeichnet, daß die Base eine Alkalimetallverbindung, vorzugsweise Natriumhydrid oder Kaliumhydrid, ist.

11. Verfahren nach einem der Ansprüche 1 bis 10, dadurch gekennzeichnet, daß das Nitroaralkylcyanid ein 2 - (4 - Nitrophenyl) - acetonitril, ein 2 - (4 - Nitrophenyl) - propionitril oder ein 2 - (3 - Halogen - 4 - nitrophenyl) - propionitril ist.

12. 2 - (3 - Fluor - 4 - nitrophenyl) - propionitril.

13. Verfahren zur Herstellung von 2 - [4 - (1,3 - Dihydro - 1 - oxo - 2H - isoindol - 2 - yl) - phenyl] - propionsäure, dadurch gekennzeichnet, daß man entweder

(a) 2 - (4 - Nitrophenyl) - propionitril nach einem Verfahren gemäß Anspruch 11 herstellt und das hergestellte 2 - (4 - Nitrophenyl) - propionitril in 2 - (4 - Aminophenyl) - propionsäure entweder durch Hydrolysieren des 2 - (4 - Nitrophenyl) - propionitrils zu 2 - (4 - Nitrophenyl) - propionsäure und Reduzieren der Säure, oder durch Reduzieren des 2 - (4 - Nitrophenyl) - propionitrils zu 2 - (4 - Aminophenyl) - propionitril und Hydrolysieren des 2 - (4 - Aminophenyl) - propionitrils, überführt, in beiden Fällen anschließend die 2 - (4 - Aminophenyl) - propionsäure mit Phthalsäureanhydrid unter Bildung von 2 - (4 - Phthalimidophenyl) - propionsäure umsetzt und die 2 - (4 - Phthalimidophenyl) - propionsäure reduziert, oder

(b) 2 - (4 - Nitrophenyl) - propionitril gemäß eine Verfahren nach Anspruch 11 herstellt und das hergestellte 2 - (4 - Nitrophenyl) - propionitril zu 2 - (4 - Aminophenyl) - propionitril reduziert, das 2 - (4 - Aminophenyl) - propionitril mit Phthalsäureanhydrid unter Bildung von 2 - (4 - Phthalimidophenyl) - propionitril umsetzt und das 2 - (4 - Phthalimidophenyl) - propionitril in 2 - [4 - (1,3 - Dihydro - 1 - oxo - 2H - isoindol - 2 - yl) - phenyl] - propionsäure entweder durch Hydrolysieren des 2 - (4 - Phthalimidophenyl) - propionitrils zu 2 - (4 - Phthalimidophenyl) - propionsäure und Reduzieren der Säure, oder durch Reduzieren des 2 - (4 - Phthalimido - phenyl) - propionitrils zu 2 - [4 - (1,3 - Dihydro - 1 - oxo - 2H - isoindol - 2 - yl) - phenyl] - propionitril mit anschließender Hydrolyse, überführt.

14. Verfahren zur Herstellung von 2 - [3 - Chlor - 4 - (3 - pyrrolinyl) - phenyl] - propionsäure, dadurch gekennzeichnet, daß man 2 - (3 - Chlor - 4 - nitrophenyl) - propionitril nach einem Verfahren gemäß Anspruch 11 herstellt und das 2 - (3 - Chlor - 4 - nitrophenyl) - propionitril zu 2 - (4 - Amino - 3 - chlorphenyl) - propionitril reduziert, das 2 - (4 - Amino - 3 - chlorphenyl) - propionitril mit einem 1,4 - Dihalogen - 2 - buten unter Bildung von 2 - [3 - Chlor - 4 - (3 - pyrrolinyl) - phenyl] - propionitril umsetzt und das 2 - [3 - Chlor - 4 - (3 - pyrrolinyl) - phenyl] - propionitril hydrolysiert.

15. Verfahren zur Herstellung von 2 - (2 - Fluor - 4 - biphenylyl) - propionsäure, dadurch gekennzeichnet, daß man 2 - (3 - Fluor - 4 - nitrophenyl) - propionitril nach einem Verfahren gemäß Anspruch 11 herstellt, das 2 - (3 - Fluor - 4 - nitrophenyl) - propionitril zu 2 - (4 - Amino - 3 - fluorphenyl) - propionitril reduziert und das hergestellte 2 - (4 - Amino - 3 - fluorphenyl) - propionitril in 2 - (2 - Fluor - 4 - biphenylyl) - propionitril mittels einer Gomberg-Bachmann-Reaktion mit Benzol umwandelt und das 2 - (2 - Fluor - 4 - biphenylyl) - propionitril hydrolysiert.

16. Verfahren zur Herstellung einer pharmazeutischen oder veterinärmedizinischen Formulierung, die als aktive Verbindung 2 - (2 - Fluor - 4 - biphenylyl) - propionsäure, 2 - [3 - Chlor - 4 - (3 - pyrrolinyl) - phenyl] - propionsäure oder 2 - [4 - (1,3 - Dihydro - 1 - oxo - 2H - isoindol - 2 - yl) - phenyl] - propionsäure enthält, dadurch gekennzeichnet, daß man die aktive Verbindung nach einem Verfahren gemäß Anspruch 15, Anspruch 14 bzw. Anspruch 13 herstellt und die aktive Verbindung für pharmazeutische oder veterinärmedizinische Verwendung formuliert.

17. Verwendung eines Verfahren gemäß Anspruch 15, Anspruch 14 bzw. Anspruch 13 zur Herstellung von 2 - (2 - Fluor - 4 - biphenylyl) - propionsäure, 2 - [3 - Chlor - 4 - (3 - pyrrolinyl) - phenyl] - propionsäure oder 2 - [4 - (1,3 - Dihydro - 1 - oxo - 2H - isoindol - 2 - yl) - phenyl] - propionsäure für eine Verwendung als Arzneimittel.

**Revendications**

1. Procédé de préparation d'un cyanure de nitro-aralkyle, caractérisé en ce qu'il consiste à faire réagir un composé nitro-aromatique avec un composé cyanure de formule

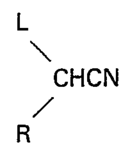

dans laquelle L représente un atome de chlore ou un atome de brome et R représente un groupe alkyle, dans un solvant aprotique pratiquement anhydre et en présence d'une base de telle sorte que le cyanure subisse une substitution nucléophile sur un atome de carbone cyclique non substitué du composé nitro-aromatique, substitution au cours de laquelle l'atome de chlore ou l'atome de brome L fonctionne comme groupe qui s'éloigne.

2. Procédé selon la revendication 1, caractérisé en ce que le composé nitro-aromatique est exempt d'halogène sur le noyau aromatique comportant le groupe nitro.

3. Procédé selon la revendication 1, caractérisé en ce que le composé nitro-aromatique est un composé halo-nitro-aromatique comportant un substituant halo sur le noyau aromatique comportant le groupe nitro.

4. Procédé selon la revendication 2 ou 3, caractérisé en ce que le composé nitro-aromatique est un mono-nitrobenzène comportant une position para non substituée vis-à-vis du groupe nitro.

5. Procédé selon la revendication 4, caractérisé en ce que le mono-nitrobenzène est le nitrobenzène ou un o-halonitrobenzène.

6. Procédé selon la revendication 1, caractérisé en ce que le composé nitro-aromatique est un chloronitrobenzène, de préference, l'o-chloronitrobenzène.

7. Procédé selon la revendication 1, caractérisé en ce que le composé nitro-aromatique est un fluoronitrobenzène, de préference, l'o-fluoronitrobenzène.

8. Procédé selon l'une quelconque des revendications 1 à 7, caractérisé en ce que R est un groupe alkyle en $C_1$—$C_{10}$.

9. Procédé selon l'une quelconque des revendications 1 à 8, caractérisé en ce que le solvant est un solvant aprotique dipolaire.

10. Procédé selon l'une quelconque des revendications 1 à 9, caractérisé en ce que la base est un composé d'un métal alcalin, de préférence, l'hydrure de sodium ou l'hydrure de potassium.

11. Procédé selon l'une quelconque des revendications 1 à 10, caractérisé en ce que le cyanure de nitro-aralkyle est un 2 - (4 - nitrophényl)acétonitrile, un 2 - (4 - nitrophényl)propionitrile ou un 2 - (3 - halo - 4 - nitrophényl)propionitrile.

12. Le 2 - (3 - fluoro - 4 - nitrophényl)propionitrile.

13. Procédé de préparation d'acide 2 - [4 - (1,3 - dihydro - 1 - oxo - 2H - isoindol - 2 - yl)phényl]propionique, caractérisé en ce que:

(a) on prepare le 2 - (4 - nitrophényl)propionitrile par un procédé selon la revendication 11 et on

convertit le 2 - (4 - nitrophényl)propionitrile préparé en acide 2 - (4 - aminophényl)propionique en hydrolysant le 2 - (4 - nitrophényl)propionitrile en acide 2 - (4 - nitrophényl)propionique et en réduisant cet acide ou en réduisant le 2 - (4 - nitrophényl)propionitrile en 2 - (4 - aminophényl)propionitrile et en hydrolysant le 2 - (4 - aminophényl)propionitrile et, dans l'un ou l'autre cas, on fait ensuite réagir l'acide 2 - (4 - aminophényl) - propionique avec l'anhydride phtalique pour former l'acide 2 - (4 - phtalimidophényl)propionique et on réduit l'acide 2 - (4 - phtalimidophényl)propionique; ou

(b) on prépare le 2 - (4 - nitrophényl)propionitrile par un procédé selon la revendication 11 et on réduit le 2 - (4 - nitrophényl)propionitrile préparé en 2 - (4 - aminophényl)propionitrile, on fait réagir le 2 - (4 - aminophényl)propionitrile avec l'anhydride phtalique pour former le 2 - (4 - phtalimidophényl) - propionitrile et on convertit le 2 - (4 - phtalimidophényl) - propionitrile en acide 2 - [4 - (1,3 - dihydro - 1 - oxo - 2H - isoindol - 2 - yl)phényl]propionique en hydrolysant le 2 - (4 - phtalimidophényl)propionitrile en acide 2 - (4 - phtalimidophényl)propionique et on réduit cet acide, ou en réduisant le 2 - (4 - phtalimidophényl)propionitrile en 2 - [4 - (1,3 - dihydro - 1 - oxo - 2H - isoindol - 2 - yl)phényl] - propionitrile, cette réduction étant suivie d'une hydrolyse.

14. Procédé de préparation d'acide 2 - [3 - chloro - 4 - (3 - pyrrolinyl)phényl]propionique, caractérisé en ce qu'il consiste à préparer le 2 - (3 - chloro - 4 - nitrophényl)propionitrile par un procédé selon la revendication 11 et réduire le 2 - (3 - chloro - 4 - nitrophényl)propionitrile en 2 - (4 - amino - 3 - chlorophényl)propionitrile, faire réagir le 2 - (4 - amino - 3 - chlorophényl)propionitrile avec un 1,4 - dihalo - 2 - butène pour former le 2 - [3 - chloro - 4 - (3 - pyrrolinyl)phényl]propionitrile et hydrolyser le 2 - [3 - chloro - 4 - (3 - pyrrolinyl)phényl]propionitrile.

15. Procédé de préparation d'acide 2 - (2 - fluoro - 4 - biphénylyl)propionique, caractérisé en ce qu'il consiste à préparer le 2 - (3 - fluoro - 4 - nitrophényl) - propionitrile par un procédé selon la revendication 11, réduire le 2 - (3 - fluoro - 4 - nitrophényl)propionitrile en 2 - (4 - amino - 3 - fluorophényl)propionitrile et convertir le 2 - (4 - amino - 3 - fluorophényl)propionitrile préparé en 2 - (2 - fluoro - 4 - biphénylyl)propionitrile au moyen d'une réaction de Gomberg-Bachmann avec du benzène, et hydrolyser le 2 - (2 - fluoro - 4 - biphénylyl)propionitrile.

16. Procédé de préparation d'une formulation pharmaceutique ou vétérinaire comprenant, comme composé actif, l'acide 2 - (2 - fluoro - 4 - biphénylyl)propionique, l'acide 2 - [3 - chloro - 4 - (3 - pyrrolinyl)phényl] - propionique ou l'acide 2 - [4 - (1,3 - dihydro - 1 - oxo - 2H - isoindol - 2 - yl)phényl]propionique, ce procédé consistant à préparer le composé actif par un procédé selon la revendication 15, la revendication 14 ou la revendication 13 respectivement et formuler le composé actif pour une utilisation pharmaceutique ou vétérinaire.

17. Utilisation d'un procédé selon la revendication 15, la revendication 14 ou la revendication 13 respectivement pour préparer l'acide 2 - (2 - fluoro - 4 - biphénylyl)propionique, l'acide 2 - [3 - chloro - 4 - (3 - pyrrolinyl)phényl]propionique ou l'acide 2 - [4 - (1,3 - dihydro - 1 - oxo - 2H - isoindol - 2 - yl)phényl]propionique en vue de l'utiliser comme produit pharmaceutique.